(19) Europäisches Patentamt
European Patent Office
Office européen des brevets

(11) EP 2 742 946 A1

(12) **EUROPEAN PATENT APPLICATION**
published in accordance with Art. 153(4) EPC

(43) Date of publication:
**18.06.2014 Bulletin 2014/25**

(21) Application number: **12785864.5**

(22) Date of filing: **14.05.2012**

(51) Int Cl.:
***A61K 36/185*** *(2006.01)* ***A61K 31/352*** *(2006.01)*
***A61K 31/7024*** *(2006.01)* ***A61P 31/10*** *(2006.01)*

(86) International application number:
**PCT/BR2012/000136**

(87) International publication number:
**WO 2012/155226 (22.11.2012 Gazette 2012/47)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.05.2011 BR PI1102578**

(71) Applicants:
- **Biotec-Bio Tecnologia Ltda**
  **CEP-14025-230 Ribeirão Preto, SP (BR)**
- **Associacão de Ensino de Ribeirão Preto**
  **CEP.14096-900-Ribeirão Preto-SP (BR)**

(72) Inventors:
- **MASSARI, Jardel**
  **CEP-14110-000 Ribeirão Preto**
  **SP (BR)**
- **PEREIRA, Ana Maria Soares**
  **CEP: 14091-310-Ribeirão Preto**
  **SP (BR)**
- **PEREIRA, Sarazete Izidia Vaz**
  **CEP-14060-480 Ribeirão Preto**
  **SP (BR)**
- **HERNANDES, Camilla**
  **CEP-14096-630 Ribeirão Preto**
  **SP (BR)**
- **BERTONI, Bianca Waleria**
  **CEP-14090-550 Ribeirão Preto**
  **SP (BR)**
- **CONTINI, Silvia Helena Taleb**
  **CEP-14020-300 Ribeirão Preto**
  **SP (BR)**

(74) Representative: **Caldwell, Judith Margaret et al**
**Keltie LLP**
**Fleet Place House**
**2 Fleet Place**
**London EC4M 7ET (GB)**

(54) **METHOD FOR PRODUCING PYROSTEGIA VENUSTA DERIVATIVES, PYROSTEGIA VENUSTA DERIVATIVES, PHARMACEUTICAL COMPOSITIONS AND USES THEREOF**

(57) The present invention relates to a method for producing compounds derived from the plant *Pyrostegia venusta* (*P. venusta*), pharmaceutical compositions containing these compounds and the uses thereof for medicinal purposes, in particular as an antimicrobial agent.

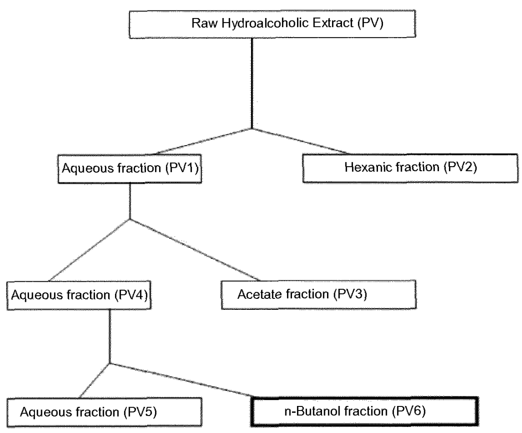


Figure 1

EP 2 742 946 A1

## Description

### Technical Field

The present invention relates to the process for producing compounds derived from the plant *Pyrostegia venusta* (*P. venusta*), pharmaceutical compositions comprising the same and its medical uses.
More particularly, the present invention relates to pharmaceutical compositions comprising, as active ingredient, standard vegetal raw extract and its fractions which especially contain mainly verbascoside, iso-verbascoside, its isomers, quercetin-3-O-α-L-rhamnopyranosyl-(1-3)-β-D-glucopyranoside and its combinations, obtained from the *Pyrostegia venusta*. Such compositions are particularly useful in antimicrobial and antioxidant treatment, especially for fighting fungal diseases caused by various species of *Candida.*

### Background of the Invention

*Pyrostegia venusta* (Ker.) Miers (synonymy *Pyrostegia ígnea and Bignonia venusta*) is a plant popularly known as são joão vine or flower. It is evergreen, woody vine, with branches of 2-4m long, blooms between the months of July and August and its flowers are very ornamental. It is a native species from Brazil, mainly occurs in sandy and poor soils and poor and is reproduced by seeds.
The flowers are used in traditional medicine to treat white spots on the body, leucoderma and vitiligo. The flower nectar is attractive to insects, since it has a high concentration of amino acids and sugars, and also contain β-sitosterol, *n*-hentriacontane, 7-O-β-D-glicopiranosilacacetina, *meso*-inositol (*myo*-inositol) and carotenoids. The *P. venusta* leaves contain stigmasterol, β-sitosterol, α-amyrin, oleanolic acid, flavonoids and phenolic compounds. In the stem bark were found triterpenes lupeol, betulin and betulinic acid and in its roots were identified allantoin, steroids, β-sitosterol, 3β-O-β-D-glucopyranosyl sitosterol and the flavanone hesperidin.
The study of Amaral et al (Evaluation of acute toxicity of angico (*Anadenanthera falcata*), rosewood (*Kilmeyera coreacea*), mastic gum (*Myracrodruon urundeuva*) and são joão vine (*Pyrostegia venusta*), by means of bioassay with *Artemia salina. Perquirere,* 2008) disclosed an acute toxicity in 48h, mentioned the authors that the use of these plants, where includes the *P. venusta,* must be performed considering its toxicological risks.
By contrast, studies of metagenesis conducted by Fernandes et al (Mutagenic test of the hydroalcoholic extract of *Pyrostegia venusta* (são joão vine) in rodent bone marrow *"in vivo",* 2008) and Magalhães et al. (Evaluation of the genotoxic potential of the raw extract of Pyrostegia venusta (Ker Gawl.) Miers, Bignoneaceae, in mouse bone marrow, 2010) showed that this hydroalcoholic leaves of *P. venusta* has no clastogenic and/or potential and/or aneugenic potential.
Although so far the presence of verbascosides in *P. venusta* has not been described in, its mixtures were isolated from the stem bark of *Arrabidaea harleyi A.H. Gentry* and was also active against microorganisms *Staphylococcus aureus, Micrococcus luteus, Bacillus subtilis, Bacillus mycoides, Enterococcus faecalis, Escherichia coli, Serratia marcensis and Candida albicans* in Disk-Diffusion tests and minimum inhibitory concentration, as reported by Lima et al., in "Antimicrobial activity of a mixture of two isomeric phenylpropanoid glycosides from Arrabidaea harleyi A.H. Gentry", (Bignoniaceae), 2003).
Thus, despite the studies cited above, until the present time nodevelopment report of antimicrobial drug from the standardized preparations, whether in the form of raw extract or purified fractions, the active ingredients of *P. venusta* is known, nor their chemical synthesis, nor the preparations of such drugs.

### Objective of the Invention

Therefore, it is the present invention object to provide a process for obtaining standard raw extract and purified fraction rich in verbascosides, from the *P. venusta.*
It is also the object of the present invention to provide standard pharmaceutical compositions containing these compounds derived from the *P. venusta* plant.
Also, it is the object of the present invention to provide pharmaceutical compositions comprising standard raw extract, purified fractions rich in verbascoside, or also combinations of the same, from the *P. venusta,* in order to act directly or indirectly as antifungal.
In an advantageous aspect of the present invention, the preparation of standard raw extract of *P. venusta,* the purified fraction rich in verbascoside and the isolation and structural characterization of molecules, as well as evaluating the pharmacological activity enabled the development of standardized pharmaceutical compositions intended for treating diseases caused by fungi.
In another advantageous aspect, the preparation of *P. venusta* extract, of the purified fraction and isolating the derivatives according to the present invention, provided facilitated processes of producing pharmaceutical compositions comprising such natural derivatives.

## Detailed Description of the Invention

According to the present invention, the process of obtaining the purified fraction rich in verbascosides, from the *P. venusta,* comprises the following basic steps producing a standard extract:

a) cold-maceration of *P. venusta* leaves with ethanol/water,
b) filtration and concentration,
c) lyophilization until the dry extract is achieved, and
d) standardization of the extract by HPLC (High-Performance Liquid Chromatography), from the standard curve.

The Figure 1 illustrates the flowchart of the raw hydroalcoholic extract (PV1) fraction of *P. venusta* until obtain the standard extract containing the verbascosides called PV6.

As evidenced from the Figure 1, *P. venusta* dry flowers were subjected to maceration with solution ethanol/water (7:3) and let in rest. After 10 days, the extract was filtered, evaporated and lyophilized to achieve a dry extract (49.0000 g). Later, this extract was dissolved in 150 ml of a solution methanol/water (2:8), before being partitioned (3x) with hexane (200 ml) followed by rota-evaporation for the complete removal of methanol, resulting an aqueous fraction PV1 (40.9776 g) and a hexanic fraction PV2 (6.5485 g). The aqueous fraction PV1 was partitioned (3x) with solution water/ethyl acetate (1:2), resulting in an acetate fraction PV3 (2.5000 g) and an aqueous fraction PV4 (37.3213 g), finally, from the said aqueous fraction PV4 another partition (3x) was performed with solution water/n-butanol (1:2), resulting in aqueous fraction PV5 (21.9000 g) and n-butanol fraction PV6 (17.600g). During division steps there was loss of about 20% in relation to the initial mass, this occurred due to the compounds impregnation in the flasks used in the extraction process and because of the low solubility of the same in the solvents used.

The standardization of the n-butanol fraction (PV6) was performed using the analytical procedure in HPLC from the standard curve established with verbascoside.

The process for producing compounds derived from the plant *P. venusta,* according to the present invention, will be described bellow with reference to one of the studies performed in the lab, whereby all recommended expectation were consolidated in the sense of obtaining verbascoside compounds useful in the antimicrobial and antioxidant treatment, specially aimed at fighting fungal diseases caused, especially, but not limited to, by several *Candida* species.

## Phytochemical Test

### Collect of plant and extract preparation:

Fresh flowers of adult plants (1.5kg) were subjected to extraction by cold-maceration process with ethanol/water (7:3) for 10 days. Thus, the extract was filtered, concentrated in rota-evaporator, and later subjected to freeze-drying until the dry extract was achieved (49g).

### Bioassay-guided fraction and isolation of chemical constituents:

The n-butanol fraction (PV6 - 15.7g) was subjected to column chromatography (Φ x h = 150 x 3.5cm) of Sephadex LH-20 and eluted with 95% ethanol and water in an increasing gradient of polarity, resulting in 16 fractions as illustrated in the table below.

Table 1 - n-butanol fraction purification

| Fractions | Eluent Solution | Volume |
|---|---|---|
| Dead volume | Ethanol 95% | 300 ml |
| 01-02 | Ethanol 95% | 50 ml each |
| 03 | Ethanol 95% | 100 ml |
| 04-13 | Ethanol 95% | 100 ml each |
| 14 | Ethanol 95% | 100 ml |
| 15 | Ethanol 95% | 200 ml |
| 16 | Water | 1000 ml |

Fractions 01 to 15 were concentrated in a rotary evaporator, chromatographed on a silica plate and pooled

according to their retention factors (Rf) into nine fractions identified in Table 2. Fraction 16 was lyophilized.

Table 2 - Grouping of the fractions by retention factor.

| Fractions | Mass (g) |
|---|---|
| PV6.1 (01-13) | 0.1966 |
| PV6.2 (04) | 0.0742 |
| PV6.3 (05-06) | 0.2526 |
| PV6.4 (07) | 0.3228 |
| PV6.5 (08) | 0.2526 |
| PV6.6 (9) | 0.4613 |
| **PV6.7 (10)** | **11.8164** |
| PV6.8 (11-15) | 0.4803 |
| PV6.9 (16) | 0.1571 |
| **TOTAL** | **13.9518** |

Yield 89,86%.

The grouped fraction PV6.7 (10g) was subjected to column chromatography (Φ x h = 170 x 2,0cm) of Sephadex LH-20 with methanol in isocratic gradient to yield 100 fractions of 6 ml each, which were compared to comparative thin layer chromatography (CCDC) and regrouped 8 new fractions.

Table 3 - Fraction PV6.7 Purification

| Fractions | Mass (g) |
|---|---|
| PV6.7.1 (01-08) | 0.1600 |
| PV6.7.2 (09-13) | 0.3100 |
| PV6.7.3 (14-18) | 1,0000 |
| **PV6.7.4 (19-20)** | **0.6449** |
| PV6.7.5 (21-24) | 1.4700 |
| **PV6.7.6 (25-38)** | **6.1391** |
| PV6.7.7 (37-54) | 0.2730 |
| PV6.7.8 (56-100) | 0.001 |
| **TOTAL** | **9.998** |

Yield: 99.98%

The fraction 6.7.4 (0.6449g) was rechromatographed in preparative plate (CCDP), resulting in 4 fractions and was subsequently subjected to purification in HPLC resulting in 3 fractions PV.6.7.4 (C1) PV6.7.4 (C2); PV6.7.4 (C3).

**PV6.7.4 (C2) Purification:**

The fraction PV6.7.4 (C2), known in the bioassays of P1 after being subjected to NMR analysis and confirmed the presence of two compounds, was again subjected to purification in HPLC, resulting in two pure molecules termed P3 and P4, which structures were determined by NMR of Hydrogen and NMR $^{13}C$.

**PV6.7.6 Purification:**

The fraction PV 6.7.6 was subjected to column chromatography (Φ x h = 170 x 2.0cm) of Sephadex LH-20 with methanol / water in isocratic gradient to yield 100 fractions of 6 ml each, which were compared by CCDC and regrouped in 6 fractions. From these six fractions, the PV.6.7.6.4 was submitted to HPLC-P, yielding 5 fractions, of which PV.6.7.6.4.4

termed P2 in biological assays has been directed to hydrogen NMR analysis and NMR $^{13}C$.

The Figure 2 illustrates a flowchart of the purification process of PV6.7.

**Results of the Phytochemical Tests**

**Structural identification of the active compounds of *P. Venusta:***

3 active compounds were identified in *P. Venusta* referenced by the codes previously described, P2, P3 and P4.

- **Iso-verbascoside (P3)**

Spectral data of $^{1}H$ NMR and $^{13}C$ (500MHZ, DMSO-d6) of the substance P1 a, compared to the values described in the literature, enabled to identify them being the iso-verbascoside structure, represented below:

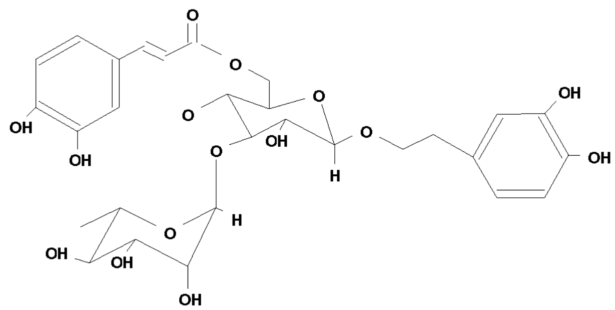

In the spectrum analysis of $^{1}H$ NMR (Table 1), it was verified the presence of signals in the region between δ 6,42-7,26, characteristic of aromatic hydrogen present in caffeoyl units and 3',4'-dihydroxyphenylethyl.

In the spectrum of $^{13}C$ NMR (Table 1), it was verified the presence of 29 signals, between them the signals in δ 146.2 and 113.9, corresponding to carbons C7"'and C8"', respectively, from the trans grouping, and in δ 168.1, corresponding to carbon C9"', present in the caffeoyl unit.

The HMQC experiment (Table 5) enabled to correlate the anomeric hydrogen signals δ 4.50 (d, *J* 7.8Hz) and 5.01 (sl), with its respective carbons C1'(δ 103.4) from glucose and C1" (δ 101.7) from rahmnose. It was also noted the correlations of hydrogen signals H3' δ 3.68 (t, *J* 9.2Hz) with C3'(δ 83.1); and H4' δ 3.55 with C4'(δ 69.4).

By analyzing the two-dimensional HMBC spectra (Table 1) it was possible to note couplings $^{3}J$ ($^{1}H$-$^{13}C$) of hydrogen H6' of glucose, with the carbonyl carbon C9"', showing that the glucose is linked to the caffeoyl group, and hydrogen in 3.68 with the carbon in δ 101.7, resulting from the binding of glucose to the H3' with the C1" from rahmnose.

Table 5 - Spectral data of HMQC and HMBC from **Vena 1** (500MHZ, $CH_3OD$-d6)

| **C/H** | **δC** | **δH** | **$^{3}J$CH** |
|---|---|---|---|
| | **$^{13}C$** | **$^{1}H$** | |
| **1** | 130.5 | - | |
| **2** | 115.6 | 6.8 (sl) | |
| **3** | 143.7 | - | |
| **4** | 145.8 | - | |
| **5** | 116.1 | 6.66 (8.1 Hz) | |
| **6** | 120.3 | 6.76 (8.1 Hz) | |
| **7** | 35.7 | 2.91 (t) | |
| **8** | 71.1 | 4.1; 3.8 | |
| **1'** | 103.4 | 4.5 (d, 7.8Hz) | H8 |
| **2'** | 74.7 | 3.44 | |
| **3'** | 83.1 | 3.68 | H1', H4' |

(continued)

| C/H | δC | δH | $^3J$CH |
|---|---|---|---|
| | $^{13}$C | $^1$H | |
| **4'** | 69.4 | 3.55 | H3' |
| **5'** | 73.0 | 3.55 | |
| **6'** | 63.6 | 4.62; 4.3 | H5' |
| **1"** | 101.7 | 5.3(sl) | H3' |
| **2"** | 71.4 | 3.44 | |
| **3"** | 71.3 | | |
| **4"** | 71.4 | | |
| **5"** | 69.0 | | |
| **6"** | 17.1 | 1.38 | |
| **1"'** | 126.7 | - | |
| **2"'** | 114.1 | 7.2(sl) | |
| **3"'** | 145.1 | - | |
| **4"'** | 148.6 | - | |
| **5"'** | 115.3 | 6.90(8.1 Hz) | |
| **6"'** | 122.1 | 7.02(8.2Hz; 1.6Hz) | |
| **7"'** | 146.2 | 7.26 (d; 15.9Hz) | |
| **8"'** | 113.9 | 6.42 (d; 15.9Hz) | |
| **9"'** | 168.1 | - | |

• **verbascoside (P2)**

The spectral data of NMR$^1$H and $^{13}$C (500MHZ, DMSO-d6) of the substance Vena 2, compared with the values described in the literature enabled to identify it as being the structure of verbascoside, which formula is presented below:

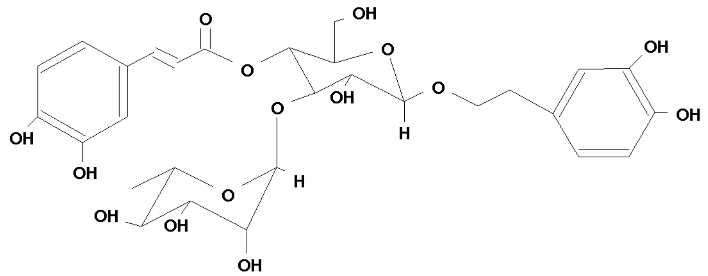

In the spectra analysis of $^1$H NMR$^1$H (Table 1), it was verified the presence of signals in the region between δ 6.20-7.44, characteristics of aromatic hydrogen, present in the caffeoyl units and 3',4'-dihydroxyphenylethyl.

In the $^{13}$C NMR$^{13}$C spectra (Table 1), it was verified the presence of 29 signals, between them the signals in δ 146.4 and 114.4, corresponding to carbons C7"'e C8"', respectively, from the trans grouping; and in δ 166.6, corresponding to carbon C9"', present in the caffeoyl unit.

The HMQC experiment (Table 1) enabled to correlate the anomeric hydrogen signals δ 4.32 (d, *J*7, 8Hz) and 5.01 (s), with its respective carbons C1'(δ 103.2) of glucose and C1" (δ 102.1) from rahmnose. It was also noted the correlation of the hydrogen signals H3' δ 3.80 (t, *J*9, 2Hz) with the C3'(δ 80.0); and H4' δ 3.35 with the C4'(δ 70.0).

By analyzing the two-dimensional HMBC spectra (Table 6) it was possible to note couplings $^3$J ($^1$H-$^{13}$C) of hydrogen H4' of glucose, with the carbonyl carbon C9"', showing that the glucose is linked to the caffeoyl group, and hydrogen in 3.69 with the carbon in δ 102.1, resulting from the binding of glucose to the H3'with the C1" from rahmnose.

Table 6 - Spectral data of HMQC and HMBC from **Vena 2** (500MHZ, DMSO-d6)

| **C/H** | δ**C** | δ**H** | $^{2}$**JCH** | $^{3}$**JCH** |
|---|---|---|---|---|
| | $^{13}$**C** | $^{1}$**H** | | |
| **1** | 130.03 | - | H7 | H8a, H8b |
| **2** | 116.6 | 6.61 (sl) | - | H7 |
| **3** | 144.40 | - | - | H5 |
| **4** | 146.4 | - | - | H2, H6 |
| **5** | 117.1 | 6.62(8.1Hz) | H6 | - |
| **6** | 120.4 | 6.49(8.1 Hz) | - | H7 |
| **7** | 35.8 | 2.69 (t) | H8a, H8b | - |
| **8** | 71.1 | 3.59(dd); 3.88(dd) | H7 | H1’ |
| **1’** | 103.2 | 4.32(d, 7.8Hz) | - | H8a, H8b |
| **2’** | 75.4 | 3.21 | H2’ | H4’ |
| **3’** | 80.0 | 3.69 | H2’, H4’ | H1" |
| **4’** | 70.0 | 4.68(t) | H3’ | - |
| **5’** | 75.4 | 3.47 | H6’ | H3’ |
| **6’** | 61.5 | 3.32- 3.39(m) | H5’ | H4’ |
| **1"** | 102.1 | 5.01 (sl) | - | H3’ |
| **2"** | 71.4 | 3.68 | H1" | H4" |
| **3"** | 71.2 | 3.28 | H2", H4" | - |
| **4"** | 72.5 | 3.1 | H2", H3" | - |
| **5"** | 69.5 | 3.35(m) | H4" | H1" |
| **6"** | 18.9 | 0.90 | - | H4" |
| **1"’** | 126.3 | - | - | H5"’, H8"’ |
| **2"’** | 155.5 | 7.01 (sl) | - | H6"’, H7"’ |
| **3"’** | 115.5 | - | - | H5"’ |
| **4"’** | 149.3 | - | - | H2"’, H6"’ |
| **5"’** | 116.3 | 6.75(8.1Hz) | - | - |
| **6"’** | 122.2 | 6.95(8.2Hz; 1.6Hz) | - | H2"’, H7"’ |
| **7"’** | 146.42 | 7.44 (d; 15.9Hz) | - | H2"’, H6"’ |
| **8"’** | 114.4 | 6.20 (d; 15.9Hz) | - | - |
| **9"’** | 167.6 | - | - | H4’, H7"’ |

• **Flavonoid (P4)**

During the $^{1}$H NMR (500MHz) analysis it was noted 5 signals in the aromatic region, which are: one doublet in δ 6.20 (1 H), with J = 2.0Hz, regarding the hydrogen H6, and other doublet in δ 6.40 (1 H), with J = 2.0 Hz, regarding the hydrogen H8 signal present in the ring A of the flavonoid. In the region between δ 6.90 and δ 7.70 of the spectrum are the signals related to hydrogen of the ring B. It was noted a doublet at δ 6.90 (1 H), regarding hydrogen H5’, which couples in ortho H6’ (J=8.0 Hz). The signal of H6’ δ 7.60 (1 H) is a double doublet (J=8.0Hz and J=2.0Hz), because it is coupling to ortho with H5’, and meta with H2’, and one doublet in δ 7.70 (J=2.0Hz), regarding the hydrogen H2’.

In addition to signals related to aglycone, two signals consistent with anomeric hydrogen were also noted. The

signal in δ 5.10 (J=5Hz) is characteristic from the anomeric hydrogen of β-D-galactose or β-D-glucose. It is know that these sugars units are distinguished only by the configuration in C4", where in the galactose the hydroxyl is in axial and in the glucose in equatorial. The appearance of the signal for C4" in δ 3.63 (sl), characteristic of the axial-equatorial coupling, leads to the conclusion that sugar is β-D-galactose.

The identification of the α-L-rhamnose, being other sugar unit, was performed with base in the anomeric hydrogen signals in δ 4.5 (sl), integrating for 1 H, and of the methyl signal in δ 1.14 (d=10Hz), integrating for 3H. It was noted, in the HMBC spectra, the correlation between anomeric hydrogen δ 5.10 and carbon in δ 134.5, confirming that the galactose is linked to the position C3 of the aglycone. It was also noted the correlation between rhamnose anomeric δ 4.50 and carbon in δ 67.5, indicating that the rhamnose is linked to the galactose carbon 6.

Based on the information obtained, compared with literature data, it was possible to identify the flavonoid as the quercetin-3-O-α-L-rhamnopyranosyl-(1→6)-β-D-glucopyranoside, from the formula:

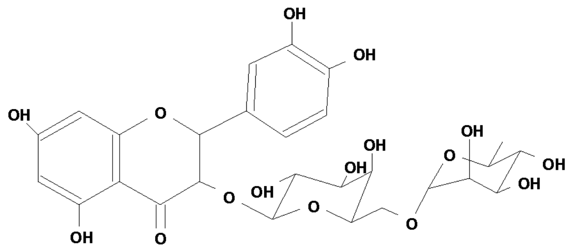

Table 7 - Spectral data of HMQC and HMBC from **Vena 1** (500MHZ, $CH_3OD$-d6)

| **C** | δ**C** |
|---|---|
| **Aglycone** | $^{13}$**C** |
| **2** | 158,2 |
| **3** | 134,5 |
| **4** | 178,3 |
| **5** | 161,9 |
| **6** | 99,1 |
| **7** | 165,6 |
| **8** | 94,0 |
| **1'** | 122,5 |
| **2'** | 116.7 |
| **3'** | 144,9 |
| **4'** | 148,7 |
| **5'** | 115,0 |
| **6'** | 122,1 |
| **Galactose** | |
| **1"** | 103,8 |
| **2"** | 71,9 |
| **3"** | 74,3 |
| **4"** | 68,7 |
| **5"** | 77,2 |
| **6"** | 67,5 |
| **Rhamnose** | |
| **1"'** | 101,4 |

(continued)

| Rhamnose | |
|---|---|
| 2"' | 71,2 |
| 3"' | 72,9 |
| 4"' | 76,2 |
| 5"' | 70.4 |
| 6"' | 16,8 |

The analysis performed in HPLC with verbascosides internal standard showed that in 1mg of the Fraction PV6 there is 498μg of verbascosides. Thus, this compound will be used as active ingredient and chemical tracer in the quality control of the drugs developed from *P. venusta.*

**Antimicrobial activity of *P. venusta* Extracts and Fractions:**

**• Test with bacteria (CLSI M7-A6- 2003):**

Preparation of the suspensions of bacterial cells

The suspensions of bacterial cells were prepared and standardized in culture medium BHI (Brain Heart Infusion - OXOID®). It was used spectrophotometer having a wavelength of 550nm and adjustment of absorbance between 0.100 and 0.125, having as white the culture medium free of inoculums or any other contaminant, corresponding to concentrations of $10^8$ CFU/mL. In this condition, the inoculum "mother" was diluted 50 times to obtain a standard inoculum in 1 to 2 x $10^4$ cells per well.

**Tests with yeast (CLSI M27-A2- 2002):**

Preparation of the suspensions of yeasts

Cultures of *Candida* incubated for 24 hours in oven at 35°C were prepared and standardized in sterile saline solution at 0.85%. The inoculum standardization was performed in a spectrophotometer equipment, having a wavelength of 530nm and absorbance adjustment between 0.125 and 0.150. Having as white the saline solution free of inoculum or any other contaminant, corresponding to the concentrations of $10^6$ CFU/mL. In this condition the inoculum "mother" was diluted 20 times and from this inoculum "child", it was performed the dilution 50 times to obtain a standard inoculum in $10^3$ cells per well.

**Test with filamentous fungi (CLSI M38-A- 2002):**

Preparation of the fungi suspension

Colonies culture of *Trichophyton rubrum* incubated for 7 days in an oven at 28 °C were harvested with a sterile spatula and then placed in conical tube covered with approximately 5 mL of saline solution at 0.9%. The resulting mixture was filtered with Whatman filter 40 (pore 8 pm) allowing only the passage of micronides, retaining hyphal fragments and then transferred to a sterile tube. The optical density was adjusted to 70 to 72% transmittance in spectrophotometer, which corresponds to $2x10^6$ to $4x10^6$ $CFU.mL^{-1}$. This suspension was diluted 1:50 in RPMI medium, which is twice the density required for test approximately $2x10^4$ to $4x10^4$ $CFU.mL^{-1}$.

Screening of the antimicrobial activity

The determination of the Minimum Inhibitory Concentration (MIC) of the raw hydroalcoholic extract and of the fractions (PV3, PV5 and PV6) was performed through microdilution test in plates containg 96 wells, according to the standards of the CLSI M27-A2 (2002) (yeasts) and CLSI (M7 A6- 2003) (bacteria). For the test it was used *Escherichia coli* -ATCC 25922, *Staphylococcus aureus* -ATCC 6538 and *Candida albicans* -ATCC 10231.

The hydroalcoholic extract (PV) was dissolved in DMSO 20% and after was dissolved in RPMI medium for test with *C. albicans and* BHI for bacteria, achieving concentrations of 2 $mg.mL^{-1}$.

**Screening of the antifungal activity from *P. venusta* Extract and Fractions:**

The determination of Minimum Inhibitory Concentration of the extracts and fractions was performed through microdilution test in plates containing 96 wells, according to standards CLSI M27-A2 (2002) (yeasts), CLSI (M38 A-2002) (filamentous fungi) with small modifications.

For the test it was used the following strains:

1. *Candida tropicalis*- USP- B3 20/08
2. *Candida tropicalis*- USP- 1658 20/08
3. *Candida parapsilosis*- ATCC 22019
4. *Candida parapsilosis*- USP- 1933 20/08
5. *Candida albicans*- ATCC 10231
6. *Candida albicans* USP- 1565-19/05/2009-20/08
7. *Candida guilhermondii*- USP-20/08
8. *Candida krusei*- ATCC 6258
9. *Candida krusei*- USP 2223-20/08
10. *Trichophyton rubrum*- ATCC MYA-3108

The hydroalcoholic extract (PV) and fractions PV3, PV5, PV6 and PV6.7 were dissolved in DMSO 20%. Ethanol leaves, acetate leaves, acetate flower, hexane flower and PV2 were dissolved in DMSO 100%. Then the samples were diluted in RPMI medium with final concentrations of 2 mg.mL $^{-1}$ for hydroalcoholic extract (PV), PV2, PV3, PV5, PV6 and PV6.7 at 1 mg.mL$^{-1}$. As a control, it was used Fluconazol® (128 $\mu$g.mL$^{-1}$), Terbinafina® (32 $\mu$g.mL$^{-1}$), Nistatina (50 $\mu$g.mL$^{-1}$) and Anfotericina B (32 $\mu$g.mL$^{-1}$).

**Antifungal activity evaluation of the Fraction PV.6.7, P1 and P2, *P venusta* semi-purified substances:**

• **Tests with yeast (LSI M27-A2- 2002)**

<u>Preparation of the samples</u>

Raw hydroalcoholic extract samples and the fraction PV6.7, dissolved previously in 20% DMSO, were diluted in RPMI medium, achieving initial concentrations of 384 $\mu$g.mL$^{-1}$. Pure substances termed P1 and P2 previously dissolved in 20% DMSO, were diluted in RPMI medium, with final concentrations of 96 $\mu$g.mL$^{-}$1. The essential oil Lima® was diluted in solution of extract flower ethanol at 384 $\mu$g.mL$^{-1}$, achieving initial concentration of 1$\mu$L.mL$^{1}$. The same was also tested individually, diluted in RPMI medium, with initial concentration of 1$\mu$L. mL$^{-1}$. As a control, it was used Cetoconazol, Miconazol and Nistatina in initial concentrations 64 $\mu$g. mL$^{-1}$ for Cetoconazol and Miconazol, and of 50 $\mu$g.mL$^{-1}$ for Nistatina.

<u>Inoculums preparation</u>

Yeasts cultures described on the Table 8 were inoculated in medium Agar-Sabouraud Dextrose and inoculated for 24 hours, at 35°C.

Table 8. Yeasts used in antimicrobial experiments

| ***Yeast*** | **Strain / Vine** |
|---|---|
| *Candida albicans* | ATCC 10231 |
| *Candida albicans* | USP 1565 |
| *Candida albicans* | USP 1 |
| *Candida albicans* | OF-M3-20 |
| *Candida albicans* | OF-M7-19 |
| *Candida krusei* | ATCC 6258 |
| *Candida krusei* | USP 2223 |
| *Candida guilhermondii* | USP |

(continued)

| *Yeast* | Strain / Vine |
|---|---|
| *Candida parapsilosis* | ATCC 22019 |
| *Candida parapsilosis* | USP 1933 |
| ***Candida tropicalis*** | USP 1658 |

Preparation of the yeasts suspensions

Cultures of *Candida sp.* were prepared and standardized in esteril saline solution at 0.85%. For inoculums standardization, a spectrophotometer equipment was used, with wavelength at 530nm and absorbance adjustment between 0.130 and 0.150, having as white the saline solution free of inoculum or any other contaminant, corresponding to concentrations of $10^6$ CFU/mL. In this condition the inoculum "mother" was diluted 50 times and from this inoculum "child" the dilution was performed 20 times to obtain a standard inoculum in $10^3$ cells per well.

The determination of Minimum Inhibitory Concentration was performed after 48 hours of incubation, as recommended by CLSI M27-A2, at 35°C. To aid in the growth view, were added to the wells dye 50μL 2,3,5-triphenyltetrazolium chloride (TTC) at 5mg.mL $^{-1}$, being performed the readings after 4 hours of incubation. Prior to the addition of dye, aliquots of 96-well plate were transferred to Petri with Agar-Sabouraud Dextrose to determine the Minimum Fungicidal Concentration. Plates were incubated for 24 hours at 35 °C.

**Antifungal activity evaluation of the Fraction PV.6.7, P1 and P2 of *P venusta:***

- **CLSI M38-A- 2002- Test with filamentous fungi**

Preparation of the samples

The samples extract flower ethanol and PV6.7, dissolved previously in 20% DMSO, were diluted in RPMI medium, achieving initial concentrations of 384pg.mL$^{-1}$. Pure substances termed P1 and P2 previously dissolved in 20% DMSO, were diluted in RPMI medium, with final concentrations of 96 μg.mL$^{-1}$. As a control, it was used Cetoconazol, Miconazol, Nistatina and Anfotericina B in initial concentrations 64 μg. mL$^{-1}$ for Cetoconazol, Miconazol and Anfotericina B and of 50 μg.mL$^{-1}$ for Nistatina.

Preparation of inoculums

Cultures of filamentous fungi described below were inoculated in Agar-Batata Dextrose medium and inoculated for 7 days, at 28°C.

| *Fungis* | Strain / Vine |
|---|---|
| *Microsporum canis* | ATCC 32903 |
| *Trichophyton menthagrophyton* | ATCC 9533 |
| *Phomopsis longicolla* | Isolado de soy |

Preparation of the fungi suspension

Colonies of cultures inoculated for 7 days in an oven at 28°C were shrouded with 0.85% saline solution, scraped and then transferred to conical tubes. After resting for 5 minutes, the supernatant was transferred to new conical tubes for subsequent quantification in spectrophotometer, at 530 nm, with the transmittance adjustment of 80-82% for *A. niger* and 70% for other microorganisms. After adjustment, there was a 1:50 dilution in RPM.

The determination of Minimum Inhibitory Concentration was performed after 7 days, at 28°C. After the incubation period, aliquots of 96-well plates were transferred to Petri plates with Potato Dextrose Agar, to determine the Minimum Fungicidal Concentration. Plates were inoculated for more 7 days, at 28°C.

The therapeutic compounds applicability evaluation of the present invention was performed in several steps and is presented below. However, it should be understood that this evaluation has no intention to limit the scope of the invention defined in the appended claims, the experiments and results.

**Antifungal activity of *P. venusta* extract incorporated in therapeutics formulation**

Preparation of the formulations

Pharmaceutical formulations in ointment, gel and cream, containing PV6 in concentrations de 0.1; 0.5; and 1.0 % (w/w) were evaluated regarding the pharmaceutical activity by diffusion method in Agar, using as positive control the antibiotics Fluconazol, Miconazol and Nistatina, in the concentration of 80 $\mu g.mL^{-1}$.

Preparation of the inoculums

Cultures of yeasts described below were inoculated in Agar-Sabouraud Dextrose medium and inoculated for 24 hours, at 35°C.

| ***Yeast*** | **Strain / Vine** |
|---|---|
| *Candida albicans* | ATCC 10231 |
| *Candida krusei* | ATCC 6258 |
| *Candida guilhermondii* | USP |
| *Candida parapsilosis* | ATCC 22019 |
| *Candida tropicalis* | USP B3 |

Preparation of the yeasts suspensions

Cultures of *Candida sp.* were prepared and standardized in esteril saline solution at 0.85%. For inoculum standardization a spectrophotometer equipment was used with wavelength at 530nm and absorbance adjustment between 0.130 and 0.150, having as white the saline solution free of inoculum or any other contaminant, corresponding to concentrations of $10^6$ CFU/mL.

Test Diffusion in Agar

In Petri plates (150x15 mm), were distributed RPMI medium with 0.8% Agar. To these plates, it was added 1 mL of the microorganism suspension in the concentration of $10^6$ CFU/mL. After inundation, the suspension excess was removed with the aid of a pipette. With the plate surface already dry, hole of approximately 6.0 mm were made, adding to each hole 20 $\mu$L of the samples and control. The plates were then incubated for 48 hours at 37°C, when halo of inhibitions were measured.

**Result of the raw hydroalcoholic extract antimicrobial evaluation:**

When tested from the bacterial strains *Escherichia coli* ATCC 25922 *and Staphylococcus aureus* ATCC 6538, the extract did not show antibacterial activity, but it was active from the strain of *Candida albicans* ATCC 10231, with MIC of 125 $\mu g.mL^{-11}$. The result is evidenced in the Table 9.

Table 9. MIC of extract flower ethanol and leave ethanol against the target strains of *screening*

| **Samples** | ***E. coli* ATCC 25922** | ***S. aureus* ATCC 6538** | ***C. albicans* ATCC 10231** |
|---|---|---|---|
| Hydroalcoholic extract | - | - | 125 $\mu g.mL^{-1}$ |
| (-) There was no growth inhibition | | | |

**Result of the antimicrobial tests from the fractions obtained from the *P. venusta* raw hydroalcoholic extract:**

When sampled from the strain of *Candida albicans* ATCC 10231, Fractions PV3 and PV6 shower lower MIC (31.25 $\mu g.mL^{-1}$), as illustrated in the Table 10. The Fraction PV6 was selected to perform the fractionating and investigation of the active substances present because show greater mass.

Table 10. MIC of Extracts and fractions against *Candida albicans* ATCC 10231.

| Samples | MIC ($\mu g.mL^{-1}$) |
|---|---|
| **Pv2** | 125 |
| **Pv3** | 31.25 |
| **Pv5** | 500 |
| **Pv6** | 31.25 |
| (-) There was no growth inhibition | |

From the extracts derived from *P. venusta* specie, the termed flower ethanol showed the lowest MIC. It was the most effective from 80% of the samples strains (8/10). In these conditions, the MIC was lower than 15.62 $\mu g.mL^{-1}$ in 87.5% of the sensitive strains. From the sampled fractions, the Pv6.7 was the most promising because shows the minor MIC from all sampled strains, according to Table 11 below.

Table 11: MIC of the extracts and fractions ($\mu g.mL^{-1}$) of *P. venusta*

| **Samples** | ***C. Tropicalis USP B3*** | ***C. Tropicalis USP 1658*** | ***C. parapsilosis ATC 22019*** | ***C. parapsilosis USP 1933*** | ***C. Albicans ATCC 10231*** | ***C. Albicans USP 1565*** | ***C. guilhermondii USP*** | ***C. krusei ATCC 6258*** | ***C. krusei ATCC 2223*** | ***Trichophyton rubrum ATCC MYA-3108*** |
|---|---|---|---|---|---|---|---|---|---|---|
| ***hydroalcoholic Extract (PV)*** | **<15.62** | **<15.62** | * | **<15.62** | **62.5** | **<15.62** | **<15.62** | **<15.62** | **<15.62** | * |
| **Pv2** | **250** | **62.5** | **31.25** | **62.5** | **125** | **15.62** | **500** | **15.62** | **31.25** | **250** |
| **Pv3** | **<7.81** | **<7.81** | **<7.81** | **<7.81** | **31.25** | **<15.62** | **<7.81** | **<7.81** | **<7.81** | **125** |
| **Pv5** | **<15.62** | **31.25** | **<15.62** | **<15.62** | **125** | **<15.62** | **<15.62** | **<15.62** | **<15.62** | **500** |
| **Pv6** | **<7.81** | **<7.81** | **<7.81** | **<7.81** | **15.62** | **15.62** | **<7.81** | **<7.81** | **<7.81** | **125** |
| **Pv6.7** | **<7.81** | **15,62** | * | **<7.81** | **<7.81** | **<7.81** | **<7.81** | **<7.81** | **<7.81** | **250** |
| **Fluconazol®** | **1** | **<1** | * | **<1** | * | **<1** | **<1** | **8** | **64** | **16** |
| **Terbinafina®** | - | - | * | - | * | - | - | - | - | **0.25** |
| **Nistalina** | **6.25** | **6.25** | * | **6.25** | * | **6.25** | **3.125** | **6.25** | **6.25** | **3.125** |
| **AnfotericinaB** | **2** | **1** | * | **2** | * | **1** | **2** | **1** | | **1** |

(*)There was no plate dryness
(-) There was no growth inhibition

The results presented in the Table 12 show that the Fraction PV.6.7 and pure compound verbascosideo (P2) are strong antifungal.

Table 12. Determining MIC (μg.mL$^{-1}$ and μL.mL$^{-1}$) from *P. venusta* fractions

| ***Yeast*** | ***Raw hydroalco holic Extract*** | ***Pv6.7*** | ***P1 (P3+P4)*** | ***P2*** | ***Cetoconazol*** | ***Miconazol*** | **Nistatina** |
|---|---|---|---|---|---|---|---|
| *C. albicans* ATCC 10231 | 24 | 3 | 12 | 3 | 32 | 4 | n.d |
| *C. albicans* USP 1565 | 0.75 | <0.75 | 0.75 | 0.375 | <0.125 | <0.125 | 6,25 |
| *C. albicans* USP 1 | 3 | <0.75 | 0.75 | 0.375 | <0.125 | <0.125 | 6,25 |
| *C. albicans* OF M3-20 | 3 | <0.75 | 0.75 | 0.375 | <0.125 | <0.125 | 12,5 |
| *C. albicans* OF M7-19 | 6 | <0.75 | 0.75 | 0.375 | <0.125 | <0.125 | 12,5 |
| *C. krusei* ATCC 6258 | 0.75 | <0.75 | 0.75 | 0.375 | <0.125 | *<1 | 6,25 |
| *C. krusei* USP 2223 | 3 | <0.75 | 0.75 | 0.75 | 2 | 1 | 6,25 |
| *C. guilhermondii* USP | 0.75 | <0.75 | 0.75 | 0.375 | 0.125 | 2 | 6,25 |
| *C. parapsilosis* ATCC 22019 | 6 | 0.75 | 1,5 | 0.375 | <0.125 | 1 | 6,25 |
| *C. parapsilosis* USP 1933 | 3 | <0.75 | 0.75 | 0.75 | <0.125 | 1 | 12,5 |
| ***C. tropicalis* USP 1658** | 3 | 0.75 | 1,5 | 1,5 | *<4 | *<2 | 6,125 |
| n.d. = not determined in the sampled concentrations<br>* = disagreeing results between triplicates | | | | | | | |

The Table 13 data demonstrated that the best antifungal results were also obtained with the Fractions Pv.6.7 and P2.

Table 13. Determining MIC (μg.mL$^{-1}$ and μL.mL$^{-1}$) from *P. venusta* samples and compounds isolated and comparison with reference antibiotics.

| ***Fungi*** | ***Raw hydroalco holic Extract*** | ***Pv6.7*** | ***P1 (P3+P4)*** | ***P2*** | ***Ceto cona zol*** | ***Micona zol*** | ***Nista tina*** | **Anf. B** |
|---|---|---|---|---|---|---|---|---|
| *Microsporum canis* ATCC 32903 | 96 | 12 | n.d | 12 | 4 | 2 | 12,5 | 1,56 |
| *Trichophyton menthagrophyton* ATCC 9533 | n.d | 48 | n.d | 48 | 2 | 2 | 12,5 | 4 |
| ***Phomopsis longicolla*-soy** | 96 | 48 | 24 | 16 | <0.125 | <0.125 | 3,125 | 0.5 |
| n.d. = not determined in the sampled concentrations. | | | | | | | | |

Table 14. MIC and CFM (μg.mL$^{-1}$) of *P. venusta* raw extract and pure substances from *Candida SP.* Strains

| Microorganism | Pv6 | | Verbascoside (P2) | | Isoverbascoside+ Flavonoid (P1) | | Isoverbascoside (P3) | | Flavonoid (P4) | | Miconazol | |
|---|---|---|---|---|---|---|---|---|---|---|---|---|
| | MIC | CFM | MIC | CFM | MIC | CFM | MIC | CFM | MIC | CFM | MIC | CFM |
| *C. krusei ATCC* | 1.5 | 6 | 1.5 | Nd | 3 | Nd | 1.5 | 12 | 6 | Nd | 1 | Nd |
| *C. krusei USP 2223* | 1.5 | Nd | 1.5 | Nd | 3 | Nd | 1.5 | Nd | 6 | Nd | 4 | Nd |
| *C. Albicans ATCC 10231* | 3 | Nd | 1.5 | Nd | 3 | nd | 3 | Nd | Nd | Nd | <0.5 | 1 |
| *C. Albicans USP 1* | 1.5 | Nd | 1.5 | Nd | 3 | Nd | 1.5 | Nd | 6 | Nd | 1 | 4 |
| *C. Albicans USP 2* | 1.5 | Nd | 1.5 | Nd | 3 | Nd | 3 | Nd | Nd | Nd | <0.5 | <0.5 |
| *C. Albicans OF M3-20* | 1.5 | Nd | 1.5 | Nd | 3 | Nd | 3 | Nd | Nd | Nd | <0.5 | <0.5 |
| *C. Albicans OF M7-19* | 0.75 | Nd | 0.75 | Nd | 1.5 | Nd | 0.75 | Nd | 6 | Nd | <0.5 | <0.5 |
| *C. parapsilosis* ATCC 22019 | 3 | Nd | 1.5 | Nd | | Nd | 1.5 | Nd | 12 | Nd | 1 | 1 |
| *C. parapsilosis* USP 1933 ATCC | 1.5 | Nd | 1.5 | Nd | 3 | Nd | 3 | Nd | Nd | Nd | <0.5 | <0.5 |
| *C. Tropicalis UPS B3* | 6 | Nd | 1.5 | Nd | 6 | Nd | 6 | Nd | Nd | Nd | <0.5 | nd |
| *Candida guilhermondi USPATCC* | 0.75 | Nd | 0.75 | Nd | 1.5 | Nd | 1.5 | 6 | 6 | Nd | 1 | 1 |
| *Candida glabrata ATCC* | 0.75 | Nd | 0.75 | Nd | 1.5 | Nd | 0.375 | Nd | 0.75 | Nd | <0.5 | 2 |

The Standard Extract PV6 that contains the 3 isolated compounds (P2, P3 and P4) show excellent antifungal activity, being indicated to be used in pharmaceutical formulations by showing the same inhibition level of the microorganisms than the pure compounds (Table 14).

Table 15. Halo of inhibition in ($\mu$g.mL$^{-1}$) of the pharmaceutical compositions obtained in sample by diffusion in Agar with *P. venusta* Standard extract against *Candida* strains.

| **Standard extract of *P. Venusta* (PV6 in %)** | ***Candida albicans* ATCC 10231** | ***Candida krusei* ATCC 6258** | ***Candida guilhermondii* USP** | ***Candida parapsilosis* ATCC 22019** | ***Candida tropicalis* USP B3** |
|---|---|---|---|---|---|
| Gel 0.1% | 0 | 0 | 2,8 | 2,0 | 0 |
| Gel 0.5% | 0 | 2,5 | 2,9 | 2,6 | 0 |
| Gel 1,0% | 1,0 | 2,8 | 3,5 | 3,0 | 2,0 |
| Cream 0.1% | 0 | 0 | 2,1 | 2,0 | 0 |
| Cream 0.5% | 0 | 1,3 | 3,0 | - | 1,5 |
| Cream 1,0% | 0 | 1,7 | 3,0 | - | 1,5 |
| Ointment 0.1% | 0 | 1,1 | 0 | - | 0 |
| Ointment 0.5% | 0 | 0 | 1,6 | - | 0 |
| Ointment 1,0% | 0 | 1,3 | 2,5 | 2,0 | 0 |
| Fluconazol 80 $\mu$g.mL$^{-1}$ | 2,2 | 0 | 2,5 | 2,0 | 2,9 |
| Miconazol 80 $\mu$g.mL$^{-1}$ | 2,6 | 2,4 | 1,8 | 1,9 | 2,5 |
| Nistatina 80 $\mu$g.mL$^{-1}$ | 1,5 | 1,2 | 1,1 | 1,6 | 0 |

In the experiments performed with diffusion in Agar, with standard extract (PV6) the formulation in gel at 1% was the best presenting the activity in all tested microorganisms. However, the formulations in cream and ointment also presented antifungal activity.

**Evaluation of the antioxidant activity of *P. venusta* samples:**

The antioxidant activity evaluation was performed through DPPH test.

Samples preparation

Extract flower ethanol samples and PV6.7 were dissolved in methanol in concentrations of 1 mg.mL$^{-1}$, 500 $\mu$g, mL$^{-1}$, 250 $\mu$g, mL$^{-1}$, 125 $\mu$g, mL$^{-1}$, 62,5 $\mu$g, mL$^{-1}$ and 31,25 $\mu$g, mL$^{-1}$. As control Metabissulfito and Rutin were used in the same conditions of the samples.

DPPH solution preparation

Around 0.002g of DPPH was dissolved in 50 mL of methanol. Then, it was verified the solution absorbance in spectrophotometer, in 517nm, which must be below 1.

Antioxidant test per se

In a plastic Eppendorf, it was added 950 $\mu$L of methanolic solution of DPPH and 50 $\mu$L of the sample. As white, it was used in the place of the sample 50$\mu$L of methanol. The samples were then incubated for 15 minutes at 30 °C. After this period, the solutions were transferred to cuvettes and performed the readings at 517nm, zeroing the spectrophotometer with methanol.

The results were calculated according the following formula:

$$\%inhibition = B-A / B \times 100$$, being A = sample, and B= White

**Results:**

The higher antioxidant potential from the *P. venusta* samples can be obtained with PV6.7, according to Table 16 illustrates. These results were equivalent to those of Rutin, trade product with strong antioxidant action. The fraction PV6.7, even at concentrations up to 125 $\mu g.mL^{-1}$, has a potential antioxidant above 90%.

Table 16. Antioxidant Activity Percentage

| ***Concentration*** | ***Extract*** | ***Pv6.7*** | ***Metabissulfito*** | **Rutin** |
|---|---|---|---|---|
| **1 $mg.mL^{-1}$** | 94,6 | 94,3 | 15,3 | 94,8 |
| **500 $\mu g.mL^{-1}$** | 79,9 | 93,9 | 8,63 | 93,8 |
| **250 $\mu g.mL^{-1}$** | 44,9 | 93,9 | 4,62 | 94,5 |
| **125 $\mu g.mL^{-1}$** | 26,1 | 93,9 | 1,33 | 93,7 |
| **62,5 $\mu g.mL^{-1}$** | 17,26 | 60.3 | 0 | 82,6 |
| **31,25 $\mu g.mL^{-1}$** | 9,86 | 32,1 | 0 | 37,9 |

The present invention has as additional object to provide pharmaceutical compositions comprising, as active ingredient, standard vegetal raw extract, especially verbascoside, isoverbascoside, its isomers, quercetin-3-O-α-L-rhamnopyranosyl-(1-3)-β-D-glucopyranoside and its combinations, obtained from the *Pyrostegia venusta.,* which are useful in the antimicrobial and antioxidant treatment, especially to fight fungal diseases caused by *Candidas.*

For the purposes of the present invention, "pharmaceutical composition" means all and any composition containing an active principle with prophylactic, palliative and/or curative purposes, acting to maintain and/or restore homeostasis and may be administered in topical form, parenteral, enteral and/or intrathecal.

As pharmaceutical compositions according to present invention can be presented in several formulations and, for that, incorporate the active ingredient in the range of 00.1 to 10% (w/w) and excipients pharmaceutically acceptable.

The excipients pharmaceutically acceptable suitable to use in the compositions of the present invention are those described in the specialized pharmaceutical literature and are here incorporated as reference, which can be used isolated or in its mixtures.

In a special way, but not limited to, pharmaceutical compositions according to the present invention can comprise, % by weight, 0-25% thickeners, 0-99.34% solvents, 0.10-20% surfactants, 0-2,0% preservatives, 0-45% wetting agent, 0.1-2,0% antioxidants, and 0-98% emollients.

For initial purposes of the present invention, (i) preferred thickeners are one or more selected from the group consisting of synthetic polymers as sodium carboxymethylcellulose and calcium carboxymethylcellulose; hydroxyethylcellulose and derivatives; polyvinylpyrrolidone; polymers and copolymers derived from acrylic acid and polyacrylamide; natural polymers as xanthan gum, gellan gum, carrageenan, pectin, alginate, esclerotium gum; aluminum silicate and derivatives; Agar; fatty acids; fatty alcohols and its condensates (ester and ether) from synthetic and natural source above 16 carbons; starch; synthetic and natural waxes as beeswax, candelila, carnaúba and ozokerite; polyethylene glycol ethoxylate; synthetic and natural hydrogenated oil; synthetic and natural hydrogenated oil; (ii) preferred solvents are one or more selected from the group consisting of alcohols and/or water; (iii) preferred surfactants are one or more selected from the group consisting of polyvinyl alcohol derivative; alcohols of ethoxylated lanolin; polyoxyethylene stearate; fatty ester of sorbitan; ethoxylated sorbitan of fatty ester; poloxamers; polyethylene glycols; acids (e salts); alcohols and its condensates (ester and ether) ethoxylates, propoxylated, sulfated, phosphatic and carbonated, quaternary ammonium; amide and amine derivatives; amino acid derivatives; alkylglycoside; lecithin; cholesterol; saponin derivatives; ethoxylate synthetic and natural source oils; (iv) preferred preservatives are one or more selected from the group consisting of methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid; natural source preservatives as essential oils; (v) preferred wetting agents are one or more selected from the group consisting of synthetic and natural glicols; lactic acid; (vi) antioxidants preferidos are one or more selected from the group consisting of BHA (butyl-hydroxyanisol); butylated hydroxytoluene (BHT); (ethylenediamine tetraacetic acid) EDTA disodium and tetrasodium; propyl gallate; sodium metabisulphite; natural antioxidants as tocopherols, phenolic acids, ascorbic acid and its derivatives, citric acid, lecithins and plants extracts as rosemary and lippia; (vii) emollients preferidos are one or more selected from the group consisting of vegetal oils as o sunflower oil, corn, soy, almonds, sesame; fatty

acids; fatty alcohols and its condensates (esters) from synthetic and natural source below 16 carbons; triglycerides; vegetal oils; minetal oil; solid vaseline; silicons; alcanolamide; lanolin; vegetable butter as shea, mango, murumuru cupuaçu.

Pharmaceutical compositions according to the present invention are produced in various forms of presentation, but those are preferred in gel, cream or ointment form. Accordingly, preferred compositions are exemplified below in Tables 17 to 19, where the percentages are given by weight.

Table 17 - Gel Formula

| | | |
|---|---|---|
| Active | 0.01- 10% | *P. venusta* Standard extract |
| Thickener | 0.05-10.00% | Synthetic polymers as sodium carboxymethylcellulose and calcium carboxymethylcellulose; hydroxyethylcellulose and derivatives; polyvinylpyrrolidone; polymers and copolymers derived from acrylic acid and polyacrylamide; natural polymers as xanthan gum; gellan gum; carrageenan; pectin; alginate; esclerotium gum, aluminum silicate and derivatives, Agar |
| Solvents | 1.00-99.34% | Alcohols and/or water |
| Surfactant | 0.10-20.00% | Polyvinyl alcohol derivative; alcohols of ethoxylated lanolin; polyoxyethylene stearate; fatty ester of sorbitan; ethoxylated sorbitan of fatty ester; poloxamers; polyethylene glycols; acids (and salts), alcohols and its condensates (ester and ether) ethoxylate, propoxylated, sulfated, phosphatic and carbonated, quaternary ammonium; amide and amine derivatives; amino acid derivatives; alkylglycoside, lecithin, cholesterol; saponin derivatives. |
| Preservatives | 0.00-2.00% | Methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid, natural source preservatives as essential oils |
| Wetting agent | 0.00-45.00% | Synthetic and natural glicols; lactic acid |
| Antioxidant | 0.01-2.00% | BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia |

Table 18 - Cream Formula

| | | |
|---|---|---|
| Active | 0.01- 10% | *P. venusta* Standard extract |
| Thickener | 0.10-25.00% | fatty acids, fatty alcohols and its condensates (ester and ether) from synthetic and natural source (above 16 carbons), starch, synthetic and natural waxes as beeswax, candelila, carnaúba and ozokerite, polyethylene glycol ethoxylate, synthetic and natural hydrogenated oil, Synthetic polymers as sodium carboxymethylcellulose and calcium carboxymethylcellulose; hydroxyethylcellulose and derivatives; polyvinylpyrrolidone; polymers and copolymers derived from acrylic acid and polyacrylamide; natural polymers as xanthan gum; gellan gum; carrageenan; pectin; alginate; esclerotium gum, aluminum silicate and derivatives, Agar |
| Surfactants | 0.10-20.00% | Ethoxylate synthetic and natural source oils, Polyvinyl alcohol derivative; alcohols of ethoxylated lanolin; polyoxyethylene stearate; fatty ester of sorbitan; ethoxylated sorbitan of fatty ester; poloxamers; polyethylene glycols; acids (and salts), alcohols and its condensates (ester and ether) ethoxylate, propoxylated, sulfated, phosphatic and carbonated, quaternary ammonium; amide and amine derivatives; amino acid derivatives; alkylglycoside, lecithin, cholesterol; saponin derivatives. |
| Wetting agent | 0.00-25.00% | Synthetic and natural glicols; lactic acid |

(continued)

| | | |
|---|---|---|
| Emollients | 0.00-15.00% | Vegetal oils as the sunflower oil, corn, soy, almonds, sesame; fatty acids, fatty alcohols and its condensates (esters) from synthetic and natural source (below 16 carbons), triglycerides, vegetal oils, minetal oil, solid vaseline, silicons, alcanolamide, lanolin, vegetable butter as shea, mango, murumuru, cupuaçu |
| Solvents | 1.00-99.29% | Alcohols and/or water |
| Antioxidant | 0.01-2.00% | BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia |
| Preservative | 0.00-2.00% | Methylparaben and propylparaben; benzyl alcohol; parabens; bronopol; cetrimide;, chlorobutanol; fenoxiethanol; imidazolidinyl urea; isothiazolinone; DMDM hydantoin; benzoic acid, sorbic and derivatives; dehydroacetic acid, ferulic acid, natural source preservatives as essential oils |

Table 19 - Ointment Formula

| | | |
|---|---|---|
| Active | 0.01- 10% | *P. venusta* Standard extract |
| Thickener | 0.00-25.00% | fatty acids, fatty alcohols and its condensates (ester and ether) from synthetic and natural source (above 16 carbons), synthetic and natural waxes as beeswax, candelila, carnaúba and ozokerite, synthetic and natural hydrogenated oil, polyethylene glycol ethoxylate |
| Emollients | 0.00-98.00% | sunflower oil; fatty acids, fatty alcohols and its condensates (esters) from synthetic and natural source (below 16 carbons), triglycerides, vegetal oils, minetal oil, solid vaseline, silicons, alcanolamide, lanolin, vegetable butter as shea, mango, murumuru, cupuacu |
| Antioxidant | 0.01-1.00% | BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia |
| Preservative | 0.00-1.00% | Methylparaben and propylparaben; benzyl alcohol; parabens; bronopol; cetrimide;, chlorobutanol; fenoxiethanol; imidazolidinyl urea; isothiazolinone; DMDM hydantoin; benzoic acid, sorbic and derivatives; dehydroacetic acid, ferulic acid, natural source preservatives as essential oils |

## Claims

**1.** A process for producing derivatives of *P. venusta,* **characterized in that** it comprises the basic steps of:

a) cold-maceration of fresh leaves of *P. venusta* with the solution ethanol /water,
b) filtration and concentration,
c) dehydration until achieve the dry extract,
d) solubilisation of the dry extract in methanol\water
e) division of the solution methanol\water in hexane, achieving an aqueous fraction PV1 and the hexanic fraction PV2,
f) division of the aqueous fraction PV1 with solution water/ethyl acetate, achieving an aqueous fraction PV4 and an acetate fraction PV3,
g) division of the aqueous fraction PV4 with solution water/n-butanol, achieving an aqueous fraction PV5 and a n-butanol fraction PV6, and
h) gathering of the n-butanol fraction PV6.
i) standardization of the n-butanol fraction PV6 by HPLC, from the standard curve.

2. The process for producing derivatives of *P. venusta,* according to claim 1, **characterized in that** the fractions divisions PV, PV1 and PV4 are performed 3 times and that the ratio of methanol/water(2:8)/hexane is (1,5:2), of water/ethyl acetate is (1:2) and of water/n-butanol is (1:2).

3. A derivate of *P. venusta,* achieved by the process defined in the claim 1, **characterized in that** it is the standard raw extract; the n-butanol fraction PV6 rich in verbascoside, iso-verbascoside, its isomers, quercetin-3-O-α-L-rhamnopyranosyl-(1-3)-β-D-glucopyranoside and its combinations; the mixes of both.

4. Pharmaceutical compositions, **characterized in that** it comprises as active ingredient standard raw extract of *P. venusta;* or n-butanol fraction PV6 rich in verbascoside, iso-verbascoside, its isomers, quercetin-3-O-α-L-rhamnopyranosyl-(1-3)-β-D-glucopyranoside and its combinations; or the mixes of both; and excipients pharmaceutically acceptable.

5. The pharmaceutical compositions, according to claim 4, **characterized in that** it comprises as active ingredient, from 0.01 to 10% (w/w) of standard raw extract of *P. venusta;* or n-butanol fraction PV6 rich in verbascoside, iso-verbascoside, its isomers, quercetin-3-O-α-L-rhamnopyranosyl-(1-3)-β-D-glucopyranoside and its combinations; or the mixes of both; and excipients pharmaceutically acceptable.

6. The pharmaceutical compositions, according to claim 5, **characterized in that** it comprises, as excipients pharmaceutically acceptable, % by weight, 0-25% thickeners, 0-99,34% solvents, 0.10-20% surfactants, 0-2,0% preservatives, 0-45% wetting agent, 0.1-2.0% antioxidants and 0-98% de emollients.

7. The pharmaceutical compositions, according to claim 6, **characterized in that** the thickeners are one or more selected of the group consisting of synthetic polymers as sodium carboxymethylcellulose and calcium carboxymethylcellulose; hydroxyethylcellulose and derivatives; polyvinylpyrrolidone; polymers and copolymers derivatives of the acrylic acid and polyacrylamide; natural polymers as xanthan gum, gellan gum, carrageenan, pectin, alginate, esclerotium gum; aluminum silicate and derivatives; agar; fatty acids; fatty alcohols and its condensates (ester and éter) from synthetic and natural source above 16 carbons; starch; synthetic and natural waxes as beeswax, candelila, carnaúba and ozokerite; polyethylene glycol ethoxylate; synthetic and natural hydrogenated oil; synthetic and natural hydrogenated oil; the solvents are one or more selected from the group consisting of alcohols and/or water; the surfactants are one or more selected from the group consisting of polyvinyl alcohol derivative; alcohols of ethoxylated lanolin; polyoxyethylene stearate; fatty ester of sorbitan; ethoxylated sorbitan of fatty ester; poloxamers; polyethylene glycols; acids (and salts); alcohols and its condensates (ester and ether) ethoxylates, propoxylated, sulfated, phosphatic and carbonated, quaternary ammonium; amide and amine derivatives; amino acid derivatives; alkylglycoside; lecithin; cholesterol; saponin derivatives; ethoxylate synthetic and natural source oils; the preservatives are one or more selected from the group consisting of methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid; preservatives of natural source as essential oils; the wetting agent are one or more selected from the group consisting of synthetic and natural glicols; lactic acid; the antioxidants are one or more selected from the group consisting of BHA (butyl-hydroxyanisol); butylated hydroxytoluene (BHT); (ethylenediamine tetraacetic acid) EDTA disodium and tetrasodium; propyl gallate; sodium metabisulphite; natural antioxidants as tocopherols, phenolic acids, ascorbic acid and its derivatives, citric acid, lecithins and plants extracts as rosemary and lippia; and the emollients are one or more selected from the group consisting of vegetal oils as the sunflower oil, corn, soy, almonds, sesame; fatty acids; fatty alcohols and its condensates (esters) from synthetic and natural source below 16 carbons; triglycerides; vegetal oils; minetal oil; solid vaseline; silicons; alcanolamide; lanolin; vegetable butter as shea, mango, murumuru cupuaçu.

8. The pharmaceutical compositions, according to any of the claims 4 to 7, **characterized in that** they are in gel, cream or ointment form.

9. The pharmaceutical compositions, according to claim 8, **characterized in that** they are in the gel form and comprise, % by weight: 0.01- 10% standard extract of *P. venusta;*
0.05-10.00% one or more between synthetic polymers as sodium carboxymethylcellulose and calcium carboxymethylcellulose; hydroxyethylcellulose and derivatives; polyvinylpyrrolidone; polymers and copolymers derivatives of the acrylic acid and polyacrylamide; natural polymers as xanthan gum; gellan gum; carrageenan; pectin; alginate; esclerotium gum, aluminum silicate and derivatives, agar;
1.00-99.34% alcohols and/or water;
0.10-20.00% one or more between polyvinyl alcohol derivative; alcohols of ethoxylated lanolin; polyoxyethylene

stearate; fatty ester of sorbitan; ethoxylated sorbitan of fatty ester; poloxamers; polyethylene glycols; acids (and salts), alcohols and its condensates (ester and ether) ethoxylate, propoxylated, sulfated, phosphatic and carbonated, quaternary ammonium; amide and amine derivatives; amino acid derivatives; alkylglycoside, lecithin, cholesterol; saponin derivatives;
0.00-2.00% one or more between methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid, preservatives from natural source as essential oils;
0.00-45.00% one or more between synthetic and natural glicols; lactic acid;
0.01-2.00% one or more between BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia.

**10.** The pharmaceutical compositions, according to claim 8, **characterized in that** they are in the cream form and comprise, % by weight:

0.01- 10% standard extract of *P. venusta;*
0.10-25.00% one or more between fatty acids, fatty alcohols and its condensates (ester and ether) from synthetic and natural source (above 16 carbons), starch, synthetic and natural waxes as beeswax, candelila, carnaúba and ozokerite, polyethylene glycol ethoxylate, synthetic and natural hydrogenated oil, Synthetic polymers as sodium carboxymethylcellulose and calcium carboxymethylcellulose; hydroxyethylcellulose and derivatives; polyvinylpyrrolidone; polymers and copolymers derived from the acrylic acid and polyacrylamide; natural polymers as xanthan gum; gellan gum; carrageenan; pectin; alginate; esclerotium gum, aluminum silicate and derivatives, Agar;
0.10-20.00% one or more between ethoxylate synthetic and natural source oils, polyvinyl alcohol derivative; alcohols of ethoxylated lanolin; polyoxyethylene stearate; fatty ester of sorbitan; ethoxylated sorbitan of fatty ester; poloxamers; polyethylene glycols; acids (and salts), alcohols and its condensates (ester and ether) ethoxylate, propoxylated, sulfated, phosphatic and carbonated, quaternary ammonium; amide and amine derivatives; amino acid derivatives; alkylglycoside, lecithin, cholesterol; saponin derivatives;
0.00-25.00% one or more between synthetic and natural glicols; lactic acid;
0.00-15.00% one or more between vegetal oils as the sunflower oil, corn, soy, almonds, sesame; fatty acids, fatty alcohols and its condensates (esters) from synthetic and natural source (below 16 carbons), triglycerides, vegetal oils, minetal oil, solid vaseline, silicons, alcanolamide, lanolin, vegetable butter as shea, mango, murumuru, cupuagu;
1.00-99.29% alcohol and/or water
0.01-2.00% one or more between BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia;
0.00-2.00% one or more between methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid, preservatives de natural source as essential oils.

**11.** The pharmaceutical compositions, according to claim 8, **characterized in that** they are in the ointment form and comprise, % by weight:

0.01- 10% standard extract of *P. venusta;*
0.00-25.00% one or more between fatty acids, fatty alcohols and its condensates (ester and ether) from synthetic and natural source (above 16 carbons), synthetic and natural waxes as beeswax, candelila, carnaúba and ozokerite, synthetic and natural hydrogenated oil, polyethylene glycol ethoxylate;
0.00-98.00% one or more between sunflower oil; fatty acids, fatty alcohols and its condensates (esters) from synthetic and natural source (below 16 carbons), triglycerides, vegetal oils, minetal oil, solid vaseline, silicons, alcanolamide, lanolin, vegetable butter as shea, mango, murumuru, cupuagu;
0.01-1.00% one or more between BHA (butyl-hydroxyanisol), butylated hydroxytoluene (BHT), (ethylenediamine tetraacetic acid) EDTA disodium and tetrasodium, propyl gallate, sodium metabisulphite, natural antioxidants as tocopherols, phenolic acids, ascorbic acid and its derivatives, citric acid, lecithins and plants extracts as rosemary and lippia;
0.00-1.00% one or more between methylparaben and propylparaben; benzyl alcohol; parabens; bronopol; cetrimide; chlorobutanol; fenoxiethanol; imidazolidinyl urea; isothiazolinone; DMDM hydantoin; benzoic acid, sorbic and derivatives; dehydroacetic acid, ferulic acid, natural source preservatives as essential oils.

**12.** An use of *P. venusta* derivative, achieved from the process, according to claim 1, which is the standard raw extract, the n-butanol fraction PV6 rich in verbascoside, iso-verbascoside, its isomers, quercetin-3-O-α-L-rhamnopyranosyl-(1-3)-β-D-glucopyranoside and its combinations; or the mixes of both, **characterized in that** it is for manufacturing a drug to fight fungal diseases caused by *Candidas.*

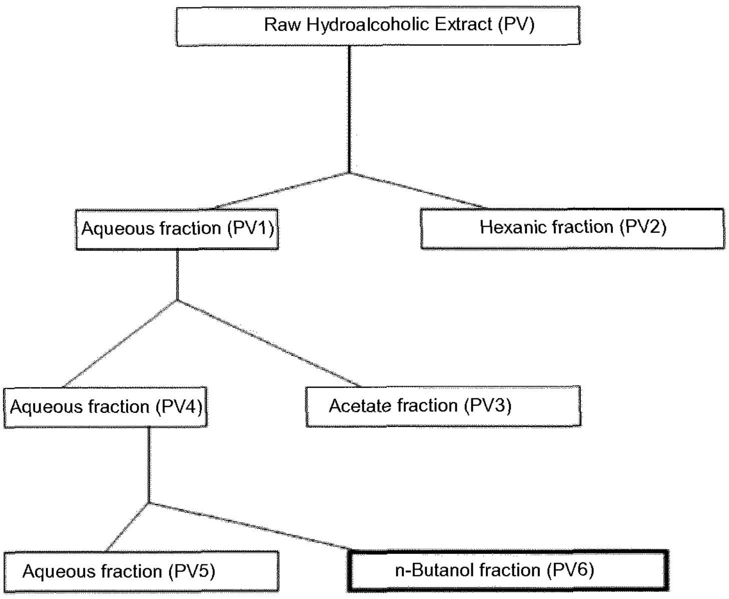


Figure 1

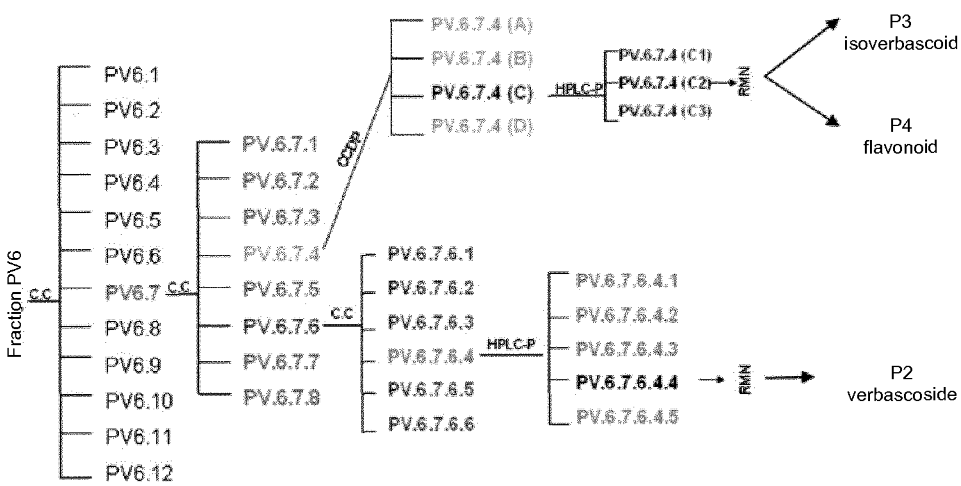


Figure 2

**INTERNATIONAL SEARCH REPORT**

International application No. PCT/BR2012/000136

| | |
|---|---|
| A. CLASSIFICATION OF SUBJECT MATTER<br>**A61K 36/185 (2006.01), A61K 31/352 (2006.01), A61K 31/7024 (2006.01), A61P 31/10 (2006.01)**<br>According to International Patent Classification (IPC) or to both national classification and IPC | |
| B. FIELDS SEARCHED | |
| Minimum documentation searched (classification system followed by classification symbols)<br>**A61K 36/185 (2006.01), A61P 31/10 (2006.01)** | |
| Documentation searched other than minimum documentation to the extent that such documents are included in the fields searched<br>**Banco de teses - CAPES, Google acadêmico** | |
| Electronic data base consulted during the international search (name of data base and, where practicable, search terms used)<br>**EPODOC, MEDLINE, WPI DATA, EMBASE, Biosis Previews, SciSearch** | |

C. DOCUMENTS CONSIDERED TO BE RELEVANT

| Category* | Citation of document, with indication, where appropriate, of the relevant passages | Relevant to claim No. |
|---|---|---|
| P,A | SILVA, PB et al. Avaliação do potencial alelopático, atividade antimicrobiana e antioxidante dos extratos orgânicos das folhas de *Pyrostegia venusta* (Ker Gawl.) Miers (Bignoniaceae). Revista Brasileira de Plantas Medicinais, 2011. Vol. 13, no. 4, pages 447-455. | |
| P,A | SOUZA FILHO, CA et al. 'Estudo fitoquímico de folhas e flores de *Pyrostegia venusta* (Bignoniaceae)'. In: 34ª Reunião Anual da Sociedade Brasileira de Química, Florianópolis, 23 a 26 may 2011. Retirado da internet: http://sec.sbq.org.br/cdrom/34ra/resumos/T1195-2.pdf<br>see the whole document | |

[X] Further documents are listed in the continuation of Box C. [ ] See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **30 july 2012** | **15.08.12** |
| Name and mailing addres INSTITUTO NACIONAL DA PROPRIEDADE INDUSTRIAL Rua Sao Bento nº 1, 17º andar cep: 20090-010, Centro - Rio de Janeiro/RJ<br>Facsimile No. +55 21 3037-3663 | Authorized officer **Giany Oliveira de Melo**<br>Telephone No. +55 21 3037-3493/3742 |

Form PCT/ISA/210 (second sheet) (July 2009)

INTERNATIONAL SEARCH REPORT

International application No.
PCT/BR2012/000136

C (Continuation). DOCUMENTS CONSIDERED TO BE RELEVANT

| Category* | Citation of document, with indication, where appropriate of the relevant passages | Relevant to claim No. |
|---|---|---|
| A | DA SILVA, PB et al. Atividade antimicrobiana e letalidade contra *Artemia salina* dos extratos obtidos a partir de *Pyrostegia venusta*. In: 30ª Reunião Anual da Sociedade Brasileira de Química. Águas de Lindóia, 31 may until 03 june 2007. Retirado da internet: https://sec.sbq.org.br/cdrom/30ra/resumos/T0309-2.pdf. see the whole document | |
| A | SOUZA FILHO, CS. Estudo fitoquímico de *Pyrostegia venusta* (Ker) Miers e de proliferação *in vitro* de melanócitos. Dissertação de Mestrado em Química. Universidade Federal do Paraná, 2010. abstract | |
| A | LEITE, KN. Contribuição ao estudo farmocognóstico de *Pyrostegia venusta* (Ker Gawl.) Miers. Dissertação de mestrado em Ciências Farmacêuticas. Universidade Estadual de Maringá, 2008. Résumo | |
| A | FERREIRA, DT et al. Constituintes químicos das raízes de *Pyrostegia venusta* e considerações sobre a sua importância medicinal. Química Nova 2000. Vol. 23, no. 1, pages 42-46 | |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **MAGALHÃES et al.** Evaluation of the genotoxic potential of the raw extract of Pyrostegia venusta (Ker Gawl.) Miers. *Bignoneaceae,* 2010
- **LIMA et al.** Antimicrobial activity of a mixture of two isomeric phenylpropanoid glycosides from Arrabidaea harleyi A.H. Gentry. *Bignoniaceae,* 2003